# EUROPEAN PATENT APPLICATION

(11) **EP 2 977 019 A1**
(43) Date of publication of application: **27.01.2016**
(21) Application number: 14769778.3
(22) Date of filing: 07.03.2014
(51) Int. Cl.: A61B 18/12, A61B 17/3211

(54) **TREATMENT TOOL**

(30) Priority: 18.03.2013 US 201361802797 P
(71) Applicant: OLYMPUS CORPORATION, Shibuya-ku Tokyo 151-0072 (JP)
(72) Inventor: SOBAJIMA, Hideo, Tokyo 151-0072 (JP); TAKASHINO, Tomoyuki, Tokyo 151-0072 (JP); TAKEI, Yusuke, Tokyo 151-0072 (JP)
(74) Representative: Schicker, Silvia
(86) International application number: PCT/JP2014/056000
(87) International publication number: WO 2014/148280

(57) **Abstract**

A treatment instrument configured to treat a living tissue by applying energy thereto includes: first and second holding members; a swing member supported by the first holding member and being rotatable in a first direction and in a second direction opposite to the first direction on a rotation axis as a supporting point; a limiting portion which limits the rotation of the swing member in one of the first direction and the second direction; a first holding surface provided on the side of the swing member close to the second holding member and configured to hold the living tissue; a second holding surface provided on the side of the second holding member close to the swing member and cooperating with the first holding surface of the swing member to hold the living tissue; and a protrusion forming a clearance between the first and second holding surfaces when the first and second holding surfaces are closed relative to each other.

## Description

### Technical Field

This invention relates to a treatment instrument for treating a living tissue by applying energy thereto.

### Background Art

For example, US 2010/0057117 A1 has disclosed a first holding member which is openable and closable relative to a second holding member (probe). The first holding member is provided with a swing member having a living tissue holding surface to hold a living tissue between the first holding member and the second holding member. Thus, the living tissue can be grasped with more uniform force between the living tissue holding surface of the swing member and the living tissue holding surface of the second holding member.

Since the swing member provided in the first holding member swings, there is a fear that the swing member or the living tissue holding surface thereof may abut on the living tissue holding surface of the second holding member.

### Summary of Invention

This invention is intended to provide a treatment instrument capable of preventing a swing member provided in a first holding member or a living tissue holding surface thereof from abutting on a living tissue holding surface of a second holding member.

According to one aspect of the present invention, a treatment instrument configured to treat a living tissue by applying energy thereto, the treatment instrument includes: first and second holding members openable and closable relative to each other, each of first and second holding members including a distal portion, a proximal portion, and a longitudinal axis defined by the distal portion and the proximal portion; a swing member supported between the distal portion and the proximal portion of the first holding member, the swing member being rotatable in a first direction and in a second direction opposite to the first direction on a rotation axis as a supporting point which extends in a direction perpendicular to the longitudinal axis and in a direction that intersects at right angles with an open-close direction of the first and second holding members; a limiting portion provided in at least one of the first holding member and the swing member and which limits the rotation of the swing member in one of the first direction and the second direction; a first holding surface which is provided on the side of the swing member close to the second holding member and which is configured to hold the living tissue; a second holding surface which is provided on the side of the second holding member close to the swing member and which faces the first holding surface and which cooperates with the first holding surface of the swing member to hold the living tissue; an energy applying portion which is provided in at least one of the first and second holding surfaces and which applies energy to the living tissue held between the first and second holding surfaces; and a protrusion having heat resisting properties and electric insulating properties which is provided in at least one of the first and second holding surfaces and which forms a clearance between the first and second holding surfaces when the first and second holding surfaces are closed relative to each other. Brief Description of Drawings
FIG. 1 is a schematic diagram showing a curative treatment system according to first and second embodiments;
FIG. 2A is a schematic diagram showing the structures of a shaft and a treatment portion of a treatment instrument according to the first embodiment, and showing how the treatment portion is closed;
FIG. 2B is a schematic diagram showing the structures of the shaft and the treatment portion of the treatment instrument according to the first embodiment, and showing how the treatment portion is opened;
FIG. 3A is a schematic top view showing a first holding member of the treatment portion of the treatment instrument according to the first embodiment;
FIG. 3B is a schematic top view showing how a swing member is attached to the first holding member of the treatment portion of the treatment instrument according to the first embodiment;
FIG. 4A is a schematic diagram showing the swing member of the treatment portion of the treatment instrument according to the first embodiment from the side of a first holding surface and a first high-frequency electrode;
FIG. 4B is a schematic longitudinal sectional view of the swing member of the treatment portion of the treatment instrument according to the first embodiment taken along the line 4B-4B in FIG. 4A;
FIG. 4C is a schematic cross sectional view of the swing member of the treatment portion of the treatment instrument according to the first embodiment taken along the line 4C-4C in FIG. 4A and FIG. 4B;
FIG. 5A is a schematic diagram showing a second holding member of the treatment portion of the treatment instrument according to the first embodiment from the side of a second holding surface and a second high-frequency electrode;
FIG. 5B is a schematic longitudinal sectional view of the second holding member of the treatment portion of the treatment instrument according to the first embodiment taken along the line 5B-5B in FIG. 5A;
FIG. 5C is a schematic cross sectional view of the second holding member of the treatment portion of the treatment instrument according to the first embodiment taken along the line 5C-5C in FIG. 5A;
FIG. 6A is a schematic diagram showing the arrangement of the first and second holding members, the swing member, a limiting portion, and a protrusion of the treatment portion of the treatment instrument according to the first embodiment;
FIG. 6B is a schematic diagram showing the arrangement of the first and second holding members, the swing member, the first and second holding surfaces, the first and second high-frequency electrodes, the limiting portion, and the protrusion of the treatment portion of the treatment instrument according to a first modification of the first embodiment;
FIG. 6C is a schematic diagram showing the arrangement of the first and second holding members, the swing member, the first and second holding surfaces, the first and second high-frequency electrodes, the limiting portion, and the protrusion of the treatment portion of the treatment instrument according to a second modification of the first embodiment;
FIG. 6D is a schematic diagram showing the arrangement of the first and second holding members, the swing member, the first and second holding surfaces, the first and second high-frequency electrodes, the limiting portion, and the protrusion of the treatment portion of the treatment instrument according to a third modification of the first embodiment;
FIG. 6E is a schematic diagram showing the arrangement of the first and second holding members, the swing member, the first and second holding surfaces, the first and second high-frequency electrodes, the limiting portion, and the protrusion of the treatment portion of the treatment instrument according to a fourth modification of the first embodiment;
FIG. 6F is a schematic diagram showing the arrangement of the first and second holding members, the swing member, the first and second holding surfaces, the first and second high-frequency electrodes, the limiting portion, and the protrusion of the treatment portion of the treatment instrument according to a fifth modification of the first embodiment;
FIG. 6G is a schematic diagram showing the arrangement of the first and second holding members, the swing member, the first and second holding surfaces, the first and second high-frequency electrodes, the limiting portion, and the protrusion of the treatment portion of the treatment instrument according to a sixth modification of the first embodiment;
FIG. 6H is a schematic diagram showing the arrangement of the first and second holding members, the swing member, the first and second holding surfaces, the first and second high-frequency electrodes, the limiting portion, and the protrusion of the treatment portion of the treatment instrument according to a seventh modification of the first embodiment;
FIG. 7 is a schematic diagram showing the arrangement of the protrusion relative to the high-frequency electrodes of the treatment portion of the treatment instrument according to the fourth to seventh modifications of the first embodiment;
FIG. 8A is a schematic diagram showing the structures of the handle and the shaft of the treatment instrument according to the second embodiment, and showing how the treatment portion is opened;
FIG. 8B is a schematic diagram showing the structures of the handle and the shaft of the treatment instrument according to the second embodiment, and showing how the treatment portion is closed;
FIG. 9A is a schematic sectional view taken along the arrow line IX-IX in FIG. 8A, showing a protective portion and a cutter driving knob of a treatment portion open-close knob of the treatment instrument according to the second embodiment;
FIG. 9B is a schematic sectional view taken along the arrow line IX-IX in FIG. 8A, showing the protective portion and the cutter driving knob of the treatment portion open-close knob of the treatment instrument according to the second embodiment;
FIG. 10A is a schematic diagram showing an interlocking member which moves together with the cutter driving knob, a drive rod, and a cutter of the treatment instrument according to the second embodiment;
FIG. 10B is a schematic diagram showing the interlocking member which moves together with the cutter driving knob of the treatment instrument according to the second embodiment;
FIG. 10C is a schematic diagram showing the cutter driving knob of the treatment instrument according to the second embodiment;
FIG. 11A is a schematic diagram showing the structure of the handle of the treatment instrument according to the first modification of the second embodiment, and showing how the treatment portion is opened;
FIG. 11B is a schematic diagram showing the structure of the handle of the treatment instrument according to the first modification of the second embodiment, and showing how the treatment portion is closed;
FIG. 12A is a schematic diagram showing the structure of the handle of the treatment instrument according to the second modification of the second embodiment, and showing how the treatment portion is opened; and
FIG. 12B is a schematic diagram showing the structure of the handle of the treatment instrument according to the second modification of the second embodiment, and showing how the treatment portion is closed.

Brief Description of Embodiments Hereinafter, embodiments of this invention will be described with reference to the drawings.

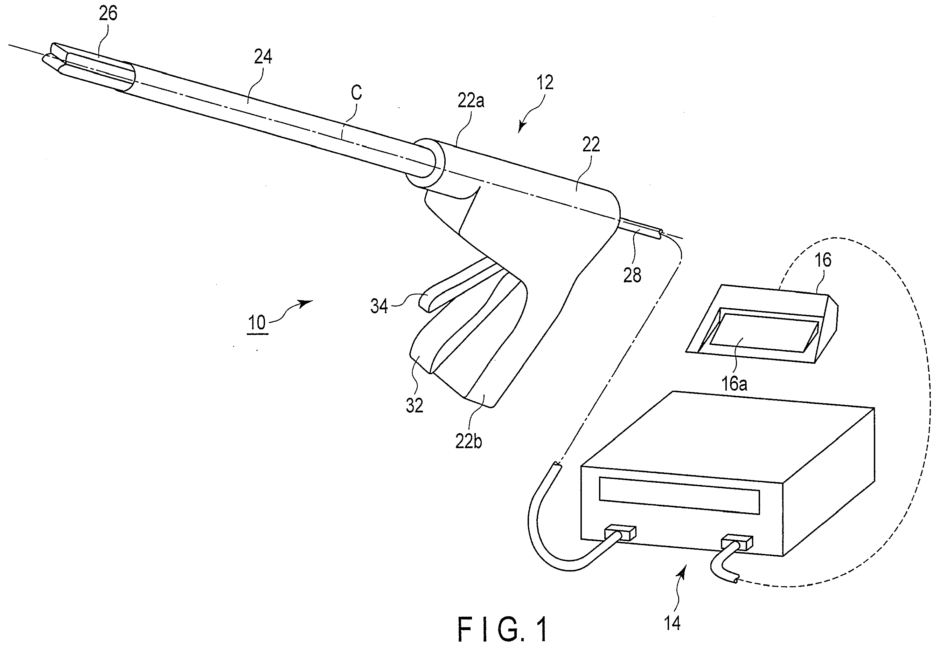

The first embodiment is described with reference to FIG. 1 to FIG. 7.

Here, a linear type bipolar treatment instrument 12 for conducting a treatment, for example, through an abdominal wall is described by way of example as a treatment instrument (energy treatment instrument) which applies energy to a living tissue to conduct a treatment.

As shown in FIG. 1, a curative treatment system 10 includes a treatment instrument (curative treatment instrument) 12, an energy source 14, and a foot switch 16 having a pedal 16a.

The treatment instrument 12 includes a handle 22, a shaft 24 having a central axis C, and a treatment portion 26. The energy source 14 is connected to the handle 22 via a cable 28. The foot switch 16 is connected to the energy source 14. A surgeon (user) operates the pedal 16a of the foot switch 16 by foot, and thereby switches on and off the supply of energy from the energy source 14 to the treatment portion 26 of the treatment instrument 12.

The handle 22 is substantially L-shaped. The shaft 24 is provided at one end (distal end) 22a of the handle 22. For example, the above-mentioned cable 28 extends from the proximal end of the handle 22 which is substantially coaxial with the shaft 24.

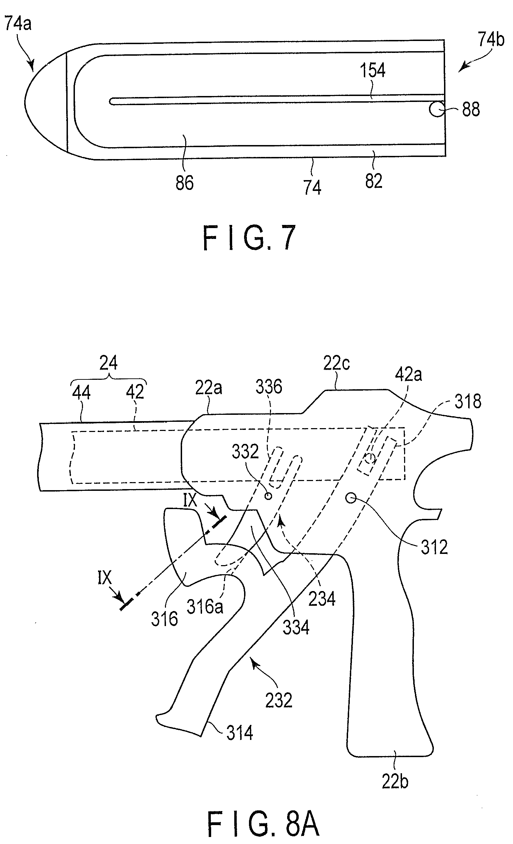

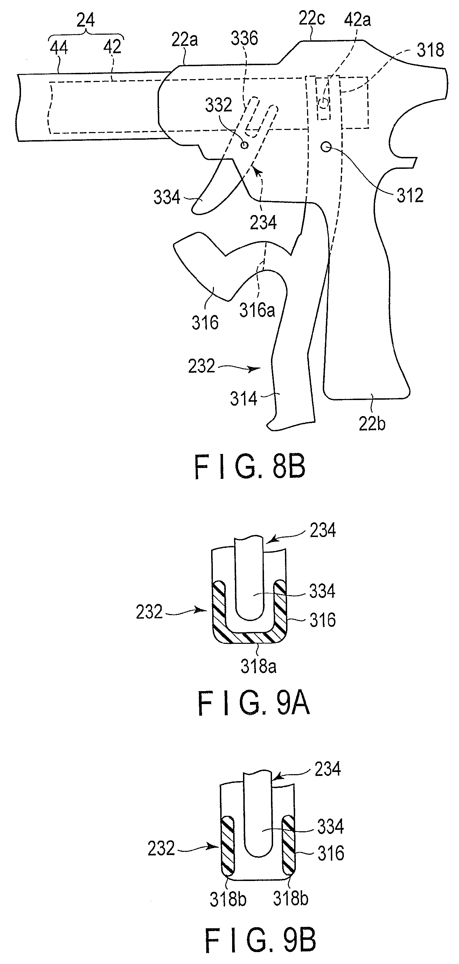

The other end 22b of the handle 22 is a grasp portion to be grasped by the surgeon. The handle 22 includes a treatment portion open-close knob (first operation body) 32 provided in parallel with the other end 22b. According to this embodiment, the treatment portion open-close knob 32 is disposed in front of the other end 22b of the handle 22. The treatment portion open-close knob 32 is rotatable inside the handle 22 by an unshown pivot shaft, that is, can be brought closer to or away from the other end of the handle 22 (see FIG. 8A and FIG. 8B). The treatment portion open-close knob 32 is coupled to the proximal end of a later-described external cylinder 44 of the shaft 24 substantially in the central part of the handle 22. Therefore, if the treatment portion open-close knob 32 is brought closer to the other end 22b of the handle 22, the later-described external cylinder 44 of the shaft 24 is advanced relative to the handle 22 along its axial direction. On the other hand, if the treatment portion open-close knob 32 is brought away from the other end 22b of the handle 22, the later-described external cylinder 44 is retreated relative to the handle 22 along its axial direction.

The handle 22 further includes a cutter driving knob (second operation body) 34 provided beside the treatment portion open-close knob 32 to move a later-described cutter 54. The cutter driving knob 34 is rotatable inside the handle 22 by an unshown pivot shaft, that is, can be brought closer to or away from the other end 22b of the handle 22 (see FIG. 8A and FIG. 8B). The cutter driving knob 34 is located in front of the treatment portion open-close knob 32 of the handle 22, and coupled to the proximal end of a later-described drive rod 52. Thus, if the cutter driving knob 34 is brought closer to the other end of the handle 22, the drive rod 52 is advanced along its axial direction, and then the later-described cutter 54 is advanced. If the cutter driving knob 34 is brought away from the other end 22b of the handle 22, the drive rod 52 is retreated along its axial direction, and then the cutter 54 is retreated.

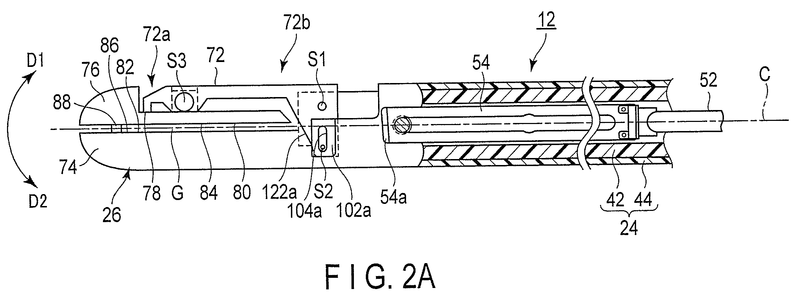

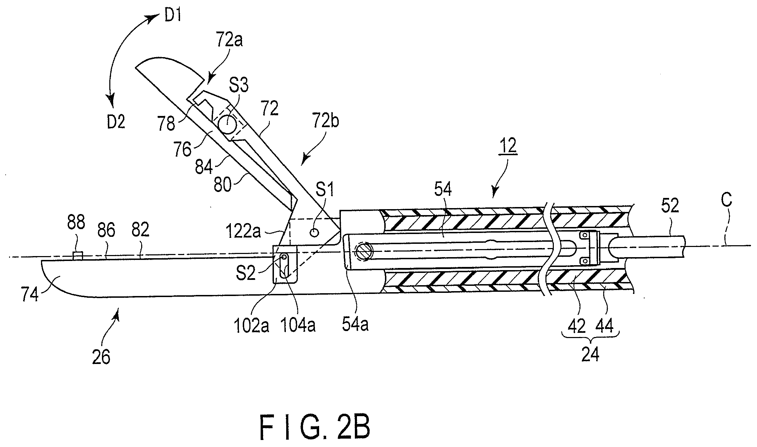

As shown in FIG. 2A and FIG. 2B, the shaft 24 includes an internal cylinder 42, and the external cylinder 44 slidably provided outside the internal cylinder 42. It is preferable that the central axis C of the internal cylinder 42 and the external cylinder 44 correspond to each other. It is preferable that the internal cylinder 42 and the external cylinder 44 have their inner circumferential surfaces and outer circumferential surfaces covered with a material having electric insulating properties. The internal cylinder 42 is fixed in its proximal portion to the handle 22. The external cylinder 44 is slidable along the axial direction of the internal cylinder 42.

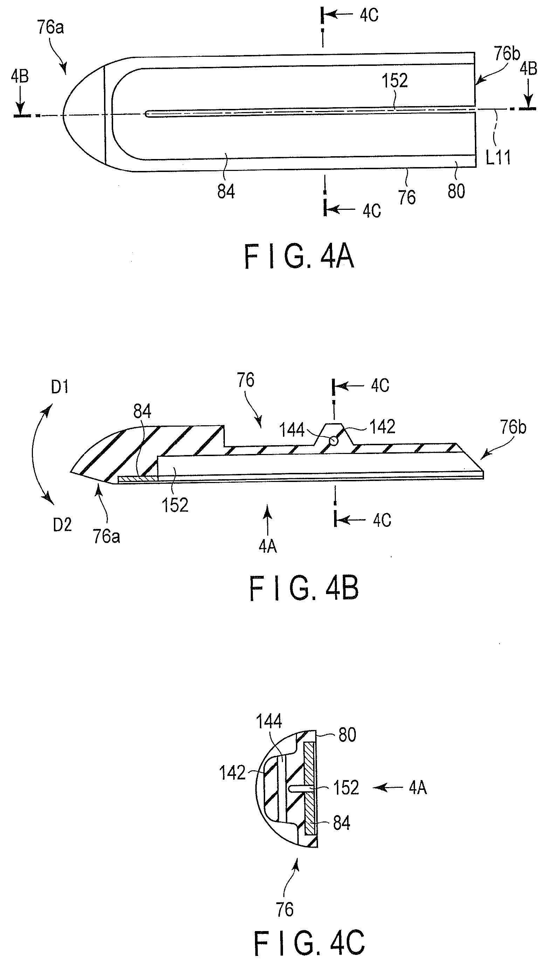

Inside (cavity portion) the internal cylinder 42 of the shaft 24, the drive rod 52 is provided movably along its axial direction. It is preferable that the central axis C of the drive rod 52 corresponds to the central axis C of the shaft 24, that is, the internal cylinder 42 and the external cylinder 44. The thin plate-shaped cutter (treatment assist tool) 54 is provided at the distal end of the drive rod 52. The cutter 54 has an edge 54a formed at its distal end. Thus, the cutter driving knob 34 is operated, so that the cutter 54 advances if the drive rod 52 is advanced, or the cutter 54 retreats if the drive rod 52 is retreated. At the same time, the cutter 54 moves along later-described first and second cutter guide grooves (flow paths, fluid discharge grooves) 152 and 154 (see FIG. 4A to FIG. 5C). Particularly the distal end of the cutter 54 is located slightly closer to the proximal side than the distal ends of the cutter guide grooves 152 and 154 when the distal end of the cutter 54 has most advanced. Depending on the degree at which a first holding member 72 is opened relative to a later-described second holding member 74 of the treatment portion 26, the distal end of the cutter 54 is set to be located inside the distal end of the internal cylinder 42 or located at the positions of the proximal ends of the cutter guide grooves 152 and 154 without contacting the living tissue when the distal end of the cutter 54 has most retreated.

As shown in FIG. 1 to FIG. 2B, the treatment portion 26 is provided at the distal end of the shaft 24. As shown in FIG. 2A and FIG. 2B, the treatment portion 26 has the first and second holding members 72 and 74, a swing member 76, a limiting portion 78, first and second holding surfaces 80 and 82, first and second energy applying portions (high-frequency electrodes) 84 and 86, and a protrusion 88.

Each of the first and second holding members 72 and 74 has electric insulating properties in at least its outer circumferential surface. Although the first holding member 72 is openable and closable relative to the second holding member 74 in the case described in this embodiment, a structure in which both the first and second holding members 72 and 74 are openable and closable relative to each other may be used. The outer surfaces of the first and second holding members 72 and 74 opposite to the first and second holding surfaces 80 and 82 are formed into smooth curved surfaces.

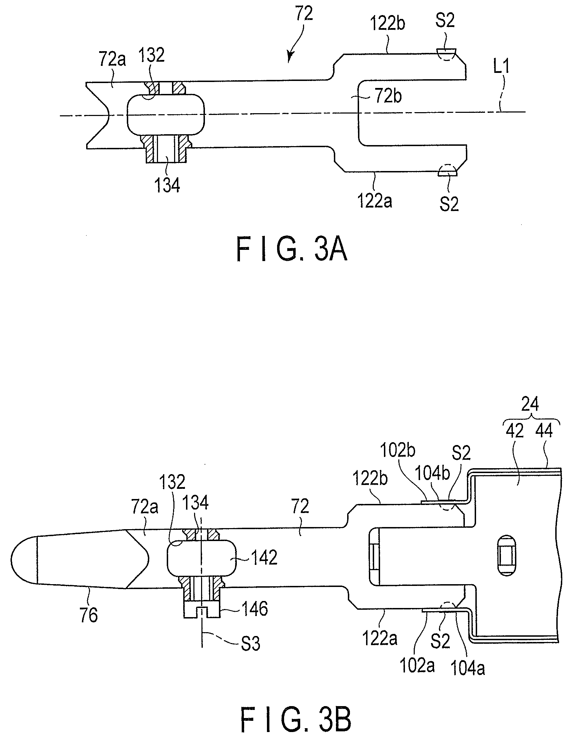

The distal end of the internal cylinder 42 has a rotation supporting point S1 which rotatably supports the proximal portion of the first holding member 72. As shown in FIG. 2A, FIG. 2B, and FIG. 3B, the distal end of the external cylinder 44 located on the outer circumference of the external cylinder 44 has a pair of planes 102a and 102b (see FIG. 3B) which respectively have long holes 104a and 104b (see FIG. 2A and FIG. 2B) formed therein and which are parallel to each other. The long holes 104a and 104b are formed to be longer, for example, in a direction that deviates from the state parallel to the central axis C. A later-described pair of arms 122a and 122b of the first holding member 72 are movably supported by the long holes 104a and 104b between the pair of planes 102a and 102b. The second holding member 74 is, for example, integrally provided at the distal end of the external cylinder 44.

The first holding member 72 has a distal portion 72a, a proximal portion 72b, and a longitudinal axis L1 defined by the distal portion 72a and the proximal portion 72b. The proximal portion 72b of the first holding member 72 shown in FIG. 2A and FIG. 2B has a pair of arms 122a and 122b in a direction that deviates from the longitudinal axis L1. The pair of arms 122a and 122b of the first holding member 72 are supported on the rotation supporting point S1 rotatably relative to the distal end of the internal cylinder 42. The pair of arms 122a and 122b have action supporting points S2 movable in the long holes 104a and 104b at the distal end of the external cylinder 44. These action supporting points S2 protrude outward from the pair of arms 122a and 122b. Thus, the action supporting points S2 of the pair of arms 122a and 122b are disposed between the planes 102a and 102b at the distal end of the external cylinder 44, and are movable in the long holes 104a and 104b of the planes 102a and 102b.

Therefore, the first holding member 72 is located at the closed position shown in FIG. 2A when the external cylinder 44 is located at a position retreated relative to the internal cylinder 42. The first holding member 72 is located at the opened position shown in FIG. 2B when the external cylinder 44 is located at a position advanced relative to the internal cylinder 42. Thus, the first holding member 72 is openable and closable relative to the second holding member 74.

As shown in FIG. 3A, the distal portion 72a of the first holding member 72 has a support recess 132 which rotatably supports the swing member 76, and a screw hole 134 which extends through the support recess 132. The screw hole 134 extends in a direction which intersects at right angles with the longitudinal axis L1 and which intersects at right angles with the open-close direction of the first and second holding members 72 and 74.

As shown in FIG. 2A, FIG. 2B, and FIG. 3B, the swing member 76 made of a material having electric insulating properties and heat resisting properties is pivotally supported by the first holding member 72 on a rotation axis S3. As shown in FIG. 4A, the swing member 76 has a distal end 76a, a proximal end 76b, and a longitudinal axis L11 defined by the distal end 76a and the proximal end 76b. The distal end 76a of the swing member 76 is located close to the distal portion 72a of the first holding member 72. The proximal end 76b of the swing member 76 is located close to the proximal portion 72b of the first holding member 72. The swing member 76 has a support projection 142 which rotates on the rotation axis S3 extending in a direction perpendicular to the longitudinal axis L1 of the first holding member 72 and the longitudinal axis L11 of the swing member 76 and in a direction that intersects at right angles with the open-close direction of the first and second holding members 72 and 74. The support projection 142 has a through-hole 144 around the rotation axis S3. The support projection 142 of the swing member 76 is fitted to the support recess 132 of the first holding member 72, and the screw hole 134 of the support recess 132 is aligned with the through-hole 144 of the support projection 142, and then a screw 146 is tightened. Thus, the swing member 76 is rotatable on the rotation axis S3 in a first direction (clockwise direction) D1 and a second direction (counterclockwise direction) D2 in FIG. 4B. The case in which the swing member 76 swings in the first direction (clockwise direction) D1 in FIG. 4B means, for example, a condition in which the distal side of the swing member 76 has risen relative to the proximal side while the first holding member 72 is closed relative to the second holding member 74. The case in which the swing member 76 swings in the second direction (counterclockwise direction) D2 in FIG. 4B means, for example, a condition in which the distal side of the swing member 76 has lowered relative to the proximal side while the first holding member 72 is closed relative to the second holding member 74. It is preferable that the rotation axis S3 of the swing member 76, the rotation supporting point S1 at the distal end of the internal cylinder 42, and the action supporting point S2 at the distal end of the external cylinder 44 are parallel to one another.

Here, as shown in FIG. 2A and FIG. 2B, the limiting portion 78 which limits the rotation of the swing member 76 in the first direction D1 is provided at a position close to the distal end 76a of the swing member 76 of the distal portion 72a of the first holding member 72 which is closer to the distal side than the rotation axis S3. Although the limiting portion 78 is formed integrally with the first holding member 72 in the case shown in this embodiment, it is also preferable that the limiting portion 78 is provided in the first holding member 72 as a separate part. The limiting portion 78 limits the rotation of the swing member 76 in the first direction (clockwise direction) D1 in FIG. 2B, and regulates the depression of the proximal side of the swing member 76 relative to the distal side.

As shown in FIG. 4B, on the side of the swing member 76 closer to the second holding member 74, the first holding surface 80 which cooperates with the later-described second holding surface 82 to grasp the living tissue is provided. Although the first holding surface 80 is formed as an outer edge of the swing member 76 in this embodiment, the first holding surface 80 is suitably changed depending on the shape and size of the first high-frequency electrode 84.

The thin plate-shaped first high-frequency electrode (first energy applying portion) 84 which applies high-frequency energy to the living tissue grasped between the first and second holding surfaces 80 and 82 to generate heat in the living tissue is fixed to the first holding surface 80. The surface of the first high-frequency electrode 84 is substantially U-shaped, and cooperates with the first holding surface 80 to form a first cutter guide groove (treatment assist tool guide groove) 152 to guide the cutter 54. That is, the first holding surface 80 and the first high-frequency electrode 84 have the linear first cutter guide groove (treatment assist tool guide groove) 152 at a position along the central axis C. It is preferable that the width of the first cutter guide groove 152 is formed to be as small as possible.

Since the first high-frequency electrode 84 is substantially U-shaped, the distal end of the first high-frequency electrode 84 is closed in the vicinity of the distal end of the swing member 76, and the proximal end is divided into two parts in the vicinity of the proximal end of the swing member 76. Thus, the inside edge of the first high-frequency electrode 84 is formed as the cutter guide groove 152. It is preferable that the rear surface and outer edge of the first high-frequency electrode 84 are covered with the swing member 76.

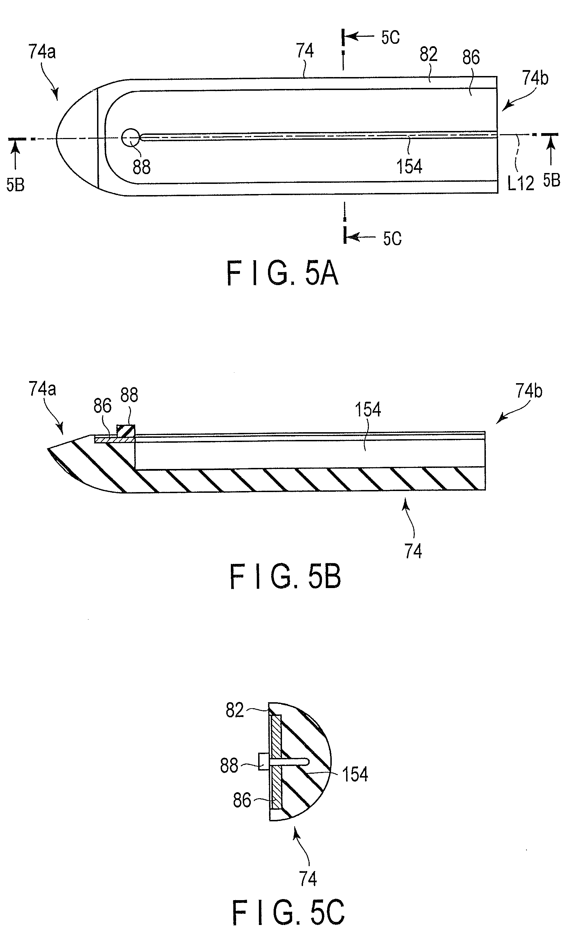

As shown in FIG. 5A and FIG. 5B, the second holding member 74 has a distal portion 74a, a proximal portion 74b, and a longitudinal axis L12 defined by the distal portion 74a and the proximal portion 74b. It is preferable that the second holding member 74 is formed integrally with the distal end of the external cylinder 44.

On the side of the second holding member 74 closer to the first holding member 72 and the swing member 76, the second holding surface 82 which cooperates with the first holding surface 80 to grasp the living tissue is provided. Although the second holding surface 82 is formed as an outer edge of the second holding member 74 in this embodiment, the second holding surface 82 is suitably changed depending on the shape and size of the second high-frequency electrode 86.

The thin plate-shaped second high-frequency electrode (second energy applying portion) 86 which applies high-frequency energy to the living tissue grasped between the first and second holding surfaces 80 and 82 to generate heat in the living tissue is fixed to the second holding surface 82. The second high-frequency electrode 86 is substantially U-shaped in the same manner as the first high-frequency electrode 84, and cooperates with the second holding surface 82 to form a second cutter guide groove (treatment assist tool guide groove) 154 to guide the cutter 54. That is, the second holding surface 82 and the second high-frequency electrode 86 have the linear second cutter guide groove (treatment assist tool guide groove) 154 at a position along the central axis C. It is preferable that the width of the second cutter guide groove 154 is formed to be as small as possible.

Since the second high-frequency electrode 86 is substantially U-shaped, the distal end of the second high-frequency electrode 86 is closed in the vicinity of the distal end of the second holding member 74, and the proximal end is divided into two parts in the vicinity of the proximal end of the second holding member 74. Thus, the inside edge of the second high-frequency electrode 86 is formed as the cutter guide groove 154. It is preferable that the rear surface and outer edge of the second high-frequency electrode 86 are covered with the second holding member 74.

The first high-frequency electrode 84 is electrically connected to the energy source 14 by an unshown conducting wire through the internal cylinder 42 or between the internal cylinder 42 and the external cylinder 44 and through the cable 28. The second high-frequency electrode 86 is electrically connected to the energy source 14 by an unshown conducting wire through the internal cylinder 42 or between the internal cylinder 42 and the external cylinder 44 and through the cable 28. Thus, if energy is supplied from the energy source 14 while the living tissue is being grasped between the first and second holding surfaces 80 and 82, that is, grasped between the first and second high-frequency electrodes 84 and 86, the living tissue grasped between the first and second high-frequency electrodes 84 and 86 can be, for example, coagulated or sealed and then treated.

It is preferable that the surface of the first high-frequency electrode 84 is formed to be longer in a direction parallel to the longitudinal axis L11 than in a direction that intersects at right angles with the longitudinal axis L11 of the swing member 76. It is preferable that the surface of the second high-frequency electrode 86 is formed to be longer in a direction parallel to the longitudinal axis L12 than in a direction that intersects at right angles with the longitudinal axis L12 of the second holding member 74. When the first and second high-frequency electrodes 84 and 86 are brought closer and then energy is applied to, for example, a tubular living tissue disposed between the first and second high-frequency electrodes 84 and 86, the living tissue between the first and second high-frequency electrodes 84 and 86 can be continuously and seamlessly sealed for the surface regions of the first and second high-frequency electrodes 84 and 86. The living tissue sealed by the application of the high-frequency energy can be separated into two parts by guiding the cutter 54 through the first and second cutter guide grooves 152 and 154. That is, for example, in the case of the tubular living tissue, the sealed part (closed part) can be cut by the cutter 54 and thereby separated into two parts.

Here, according to this embodiment, the protrusion (spacer) 88 is provided on the surface of the second high-frequency electrode 86 to keep a distance between the first high-frequency electrode 84 and the second high-frequency electrode 86 and prevent abutment while the first and second holding members 72 and 74 are closed. The protrusion 88 is made of a material having heat resisting properties and electric insulating properties. For example, a resin material such as PTFE and PEEK, ceramics, or a material such as PTFE having heat resisting properties and electric insulating properties which covers the outer periphery of a metallic material is used for the protrusion 88. The height and hardness are adjusted in consideration of the deformation of the resin material pressed by the first high-frequency electrode 84 and the living tissue when the resin material is used.

As shown in FIG. 5A and FIG. 5B, it is preferable that the protrusion 88 is located close to the distal end of the cutter guide groove 154. The protrusion 88 is provided on the same side (here, the distal side as shown in FIG. 5A and FIG. 5B) where the limiting portion 78 is provided (here, on the distal side as shown in FIG. 2B) relative to the rotation axis S3 of the swing member 76. It is also preferable that the protrusion 88 is located at a position that does not prevent the second high-frequency electrode 86 from seamlessly and continuously sealing, for example, the tubular living tissue. Thus, according to this embodiment, the protrusion 88 is located in the vicinity of the distal portion of the surface of the second high-frequency electrode 86 and located closer to its inner edge (second cutter guide groove 154) than its outer edge.

One protrusion 88 is not exclusively provided. More than one protrusion 88, for example, two protrusions 88 may be provided as long as the protrusions do not prevent the second high-frequency electrode 86 from seamlessly and continuously sealing the living tissue. In FIG. 5B, the protrusion 88 is mounted on the second high-frequency electrode 86 in a fixed state. However, it is also preferable that the protrusion 88 is fixed to the second holding member 74 through the second high-frequency electrode 86 because the protrusion 88 has heat resisting properties and electric insulating properties.

The first and second cutter guide grooves 152 and 154 are also used as fluid discharge grooves which are in communication with the inside of the internal cylinder 42 where the drive rod 52 of the cutter 54 is provided and which receive a fluid generated from the living tissue.

Therefore, the treatment portion 26 according to this embodiment has the structure described below.

The first holding member 72 has the distal portion 72a, the proximal portion 72b, and the longitudinal axis L1 defined by the distal portion 72a and the proximal portion 72b. The swing member 76 is supported between the distal portion 72a and the proximal portion 72b of the first holding member 72. The swing member 76 is rotatable in the first direction D1 and in the second direction D2 opposite to the first direction D1 on the rotation axis S3 as a supporting point which extends in a direction perpendicular to the longitudinal axis L11 and in a direction that intersects at right angles with the open-close direction of the first and second holding members 72 and 74. That is, the first holding member 72 and the swing member 76 form what is known as a seesaw jaw. The limiting portion 78 is provided in the first holding member 72 in this embodiment, and limits the rotation of the swing member 76 in the first direction D1. The first holding surface 80 is provided on the side of the swing member 76 closer to the second holding member 74, and can grasp the living tissue. The second holding surface 82 is provided on the side of the second holding member 74 closer to the swing member 76, faces the first holding surface 80, and can cooperate with the first holding surface 80 of the swing member 76 to grasp the living tissue. The first energy applying portion (first high-frequency electrode) 84 is also used as the first holding surface 80 in this embodiment, and can apply energy to the living tissue held between the first and second holding surfaces 80 and 82. The second energy applying portion (second high-frequency electrode) 86 is also used as the second holding surface 82 in this embodiment, and can cooperate with the first energy applying portion 84 to apply energy to the living tissue grasped between the first and second holding surfaces 80 and 82. The protrusion 88 is made of a material having heat resisting properties and electric insulating properties. The protrusion 88 is provided in the second holding surface 82, that is, the second energy applying portion 86 in this embodiment, and can form a clearance G (see FIG. 2A) between the first and second holding surfaces 80 and 82 when the first and second holding surfaces 80 and 82 are closed relative to each other.

The protrusion 88 faces the limiting portion 78 across the swing member 76 when the first and second holding surfaces 80 and 82 are closed relative to each other.

The first holding surface 80 has the first guide groove 152 to guide the cutter 54 capable of cutting the living tissue grasped between the first and second holding surfaces 80 and 82. The second holding surface 82 has the second guide groove 154 which cooperates with the first guide groove 152 to guide the cutter 54. The protrusion 88 is located adjacent to at least one of the first and second guide grooves 152 and 154.

The energy applying portions 84 and 86 are substantially U-shaped to have the guide grooves 152 and 154 which cooperate with at least one of the first and second holding surfaces 80 and 82 to guide the cutter 54. The protrusion 88 is located at a position in the substantially U-shaped energy applying portions 84 and 86 that does not prevent the continuous sealing of the living tissue.

Next, the function of the curative treatment system 10 according to this embodiment is described.

As shown in FIG. 2A, the treatment portion 26 is inserted into a duct line such as a body cavity while the first holding member 72 is closed relative to the second holding member 74. The treatment portion 26 is then placed to face a living tissue to be treated. The treatment portion open-close knob 32 of the handle 22 is pulled to advance the external cylinder 44 relative to the internal cylinder 42. Thus, as shown in FIG. 2B, the first holding member 72 is opened relative to the second holding member 74. In this state, the living tissue to be treated is disposed between the first and second holding surfaces 80 and 82. That is, the living tissue to be treated is disposed between the first and second high-frequency electrodes 84 and 86. In this state, the treatment portion open-close knob 32 located before the handle 22 is moved forward, and the external cylinder 44 is retreated relative to the internal cylinder 42. Thus, as shown in FIG. 2A, the first holding member 72 is closed relative to the second holding member 74. That is, the living tissue to be treated is grasped between the first and second high-frequency electrodes 84 and 86.

The swing member 76 will rotate in the counterclockwise direction D2 on the rotation axis S3 as a supporting point in FIG. 2A when the living tissue is mainly grasped in the regions of the first and second high-frequency electrodes 84 and 86 on the proximal side. At the same time, the protrusion 88 protruding from the surface of the second high-frequency electrode 86 toward the first high-frequency electrode 84 maintains the space G between the first and second high-frequency electrodes 84 and 86, and prevents the first and second high-frequency electrodes 84 and 86 from causing a short circuit.

The swing member 76 will rotate in the clockwise direction D1 on the rotation axis S3 as a supporting point in FIG. 2A when the living tissue is mainly grasped in the regions of the first and second high-frequency electrodes 84 and 86 on the distal side. At the same time, the limiting portion 78 provided at the distal end of the first holding member 72 can regulate the rotation of the swing member 76 in the clockwise direction D1. Thus, the limiting portion 78 maintains the space G between the first and second high-frequency electrodes 84 and 86, and prevents the first and second high-frequency electrodes 84 and 86 from causing a short circuit.

While the living tissue to be treated is grasped to maintain the space G between the first and second high-frequency electrodes 84 and 86 as above, the pedal 16a of the foot switch 16 is depressed by foot. Accordingly, high-frequency energy is applied to the living tissue between the first and second high-frequency electrodes 84 and 86 from the energy source 14 to generate heat in the living tissue and then coagulate the living tissue. At this point, the living tissue is coagulated regardless of the widths of the first and second cutter guide grooves 152 and 154 because the cutter guide grooves 152 and 154 are formed to be narrow. The cutter 54 is then moved along the cutter guide grooves 152 and 154 as needed to cut the treated living tissue.

As described above, the following can be said according to this embodiment.

In the treatment portion 26 of the treatment instrument 12 according to this embodiment, the swing member 76 is pivotally supported by the first holding member 72 on the rotation axis S3, and the first holding member 72 and the swing member 76 form what is known as a seesaw jaw. Thus, the amount of grasp force to grasp the living tissue between the first holding surface 80 and the first high-frequency electrode 84 of the swing member 76 and the second holding surface 82 and the second high-frequency electrode 86 of the second holding member 74 can be uniform.

When the first holding member 72 is closed relative to the second holding member 74 and the living tissue is thus grasped between the first and second high-frequency electrodes 84 and 86, for example, when the living tissue is grasped in the regions of the first and second high-frequency electrodes 84 and 86 on the proximal side, the swing member 76 rotates to press the protrusion 88 by the first high-frequency electrode 84. At this point, the protrusion 88 can prevent the first and second high-frequency electrodes 84 and 86 from causing a short circuit because the protrusion 88 has heat resisting properties and electric insulating properties. The swing member 76 rotates to press the limiting portion 78 of the first holding member 72 when the living tissue is grasped in the regions of the first and second high-frequency electrodes 84 and 86 on the distal side. At this point, the limiting portion 78 can prevent the first and second high-frequency electrodes 84 and 86 from causing a short circuit because the limiting portion 78 has heat resisting properties and electric insulating properties. In particular, in the case where the treatment portion 26 of the treatment instrument 12 has the first and second high-frequency electrodes 84 and 86 facing each other, a short circuit can be prevented when the living tissue is grasped, so that the safety of the treatment instrument 12 can be improved, and the reliability of the treatment instrument 12 can be improved.

In addition, the protrusion 88 also has an anti-slip feature, so that it is possible to inhibit the living tissue from slipping toward the distal side and proximal side of the first and second holding surfaces 80 and 82.

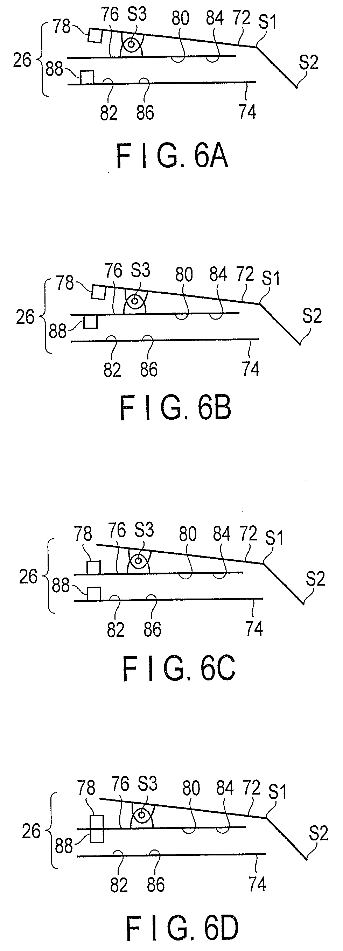

Next, modifications of this embodiment are described with reference to FIG. 6A to FIG. 7.

The relation between the first and second holding members 72 and 74, the swing member 76, the limiting portion 78, and the protrusion 88 according to this embodiment has seven modifications as schematically shown in FIG. 6B to FIG. 6H in addition to the case described in the first embodiment schematically shown in FIG. 6A.

In the example of FIG. 6A, as has been described above in the first embodiment, the limiting portion 78 is located on the side close to the distal end 76a of the swing member 76 in the distal portion 72a of the first holding member 72 which is closer to the distal side than the rotation axis S3, and the protrusion 88 is formed at a position in at least one of the surfaces of the second holding surface 82 and the second high-frequency electrode 86 close to the distal end of the second holding surface 82.

In the example of FIG. 6B, the limiting portion 78 is located on the side close to the distal end 76a of the swing member 76 in the distal portion 72a of the first holding member 72 which is closer to the distal side than the rotation axis S3, and the protrusion 88 is formed at a position in at least one of the surfaces of the first holding surface 80 and the first high-frequency electrode 84 close to the distal end of the first holding surface 80. In this modification, the position of the protrusion 88 is only changed from the second holding surface 82 to the first holding surface 80 compared to the first embodiment.

In the example of FIG. 6C, the limiting portion 78 is located on the side close to the distal portion 72a of the first holding member 72 in the distal end 76a of the swing member 76 which is closer to the distal side than the rotation axis S3, and the protrusion 88 is formed at a position in at least one of the surfaces of the second holding surface 82 and the second high-frequency electrode 86 close to the distal end of the second holding surface 82. In this modification, the position of the limiting portion 78 is only changed from the first holding member 72 to the rear surface of the first holding surface 80 of the swing member 76 compared to the first embodiment.

In the example of FIG. 6D, the limiting portion 78 is located on the side close to the distal portion 72a of the first holding member 72 in the distal end 76a of the swing member 76 which is closer to the distal side than the rotation axis S3, and the protrusion 88 is formed at a position in at least one of the surfaces of the first holding surface 80 and the first high-frequency electrode 84 close to the distal end of the second holding surface 82. In this modification, the position of the limiting portion 78 is only changed from the first holding member 72 to the rear surface of the first holding surface 80 of the swing member 76, and the position of the protrusion 88 is only changed from the second holding surface 82 to the first holding surface 80 compared to the first embodiment.

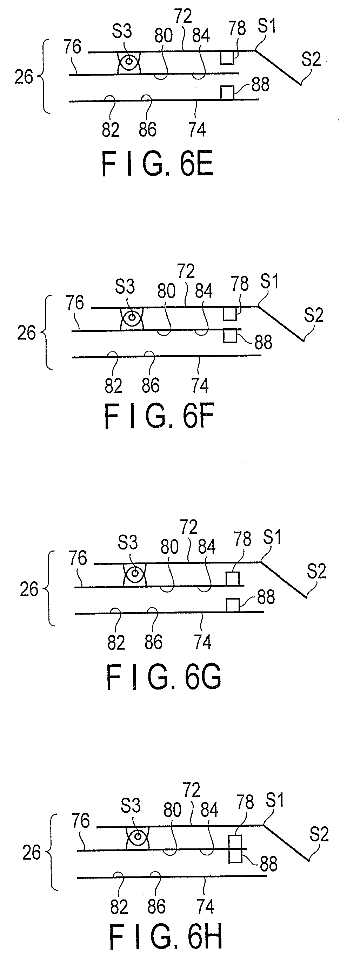

In the example of FIG. 6E, the limiting portion 78 is located on the side close to the proximal end 76b of the swing member 76 in the proximal portion 72b of the first holding member 72 which is closer to the proximal side than the rotation axis S3, and the protrusion 88 is formed at a position in at least one of the surfaces of the second holding surface 82 and the second high-frequency electrode 86 close to the proximal end of the second holding surface 82. When the living tissue is grasped on the distal sides of the first and second high-frequency electrodes 84 and 86, the swing member 76 will rotate in the first direction (clockwise direction) D1 relative to the rotation axis S3, but its rotation is prevented by the protrusion 88. On the other hand, when the living tissue is grasped on the proximal sides of the first and second high-frequency electrodes 84 and 86, the swing member 76 will rotate in the second direction (counterclockwise direction) D2 relative to the rotation axis S3, but its rotation is prevented by the limiting portion 78. Therefore, contact between the first and second high-frequency electrodes 84 and 86 is prevented.

The protrusion 88 is located at a position that least prevents the application of energy to the living tissue from the first and second high-frequency electrodes 84 and 86 as shown in FIG. 7 when the protrusion 88 is disposed on the proximal end of the first high-frequency electrode 84 or the proximal ends of the first and second high-frequency electrodes 84 and 86. For example, the protrusion 88 is formed at a position of the second high-frequency electrode 86 shown in FIG. 7 adjacent to the second cutter guide groove 154 on the inner edge of the second high-frequency electrode 86.

In the example of FIG. 6F, the limiting portion 78 is located on the side close to the proximal end 76b of the swing member 76 in the proximal portion 72b of the first holding member 72 which is closer to the proximal side than the rotation axis S3, and the protrusion 88 is formed at a position in at least one of the surfaces of the first holding surface 80 and the first high-frequency electrode 84 close to the proximal end of the first holding surface 80.

In the example of FIG. 6G, the limiting portion 78 is located on the side close to the proximal portion 72b of the first holding member 72 in the proximal end 76b of the swing member 76 which is closer to the proximal side than the rotation axis S3, and the protrusion 88 is formed at a position in at least one of the surfaces of the second holding surface 82 and the second high-frequency electrode 86 close to the proximal end of the second holding surface 82.

In the example of FIG. 6H, the limiting portion 78 is located on the side close to the proximal portion 72b of the first holding member 72 in the proximal end 76b of the swing member 76 which is closer to the proximal side than the rotation axis S3, and the protrusion 88 is formed at a position in at least one of the surfaces of the first holding surface 80 and the first high-frequency electrode 84 close to the proximal end of the first holding surface 80.

In these modifications, both the limiting portion 78 and the protrusion 88 are located on the same distal side or proximal side relative to the rotation axis S3 of the swing member 76. It is recognized that the limiting portion 78 has only to be disposed in at least one of the first holding member 72 and the swing member 76 and that the protrusion 88 has only to be disposed in at least one of the first and second holding surfaces 80 and 82.

The treatment portion 26 can obtain the same functions and advantageous effects as those described in the first embodiment when the treatment portion 26 is formed as in the modifications shown in FIG. 6B to FIG. 6H in addition to FIG. 6A which is a schematic diagram of the treatment portion 26 in the first embodiment.

Therefore, according to the first embodiment including the modifications, the limiting portion 78 is provided on the side closer to the distal portion 72a of the first holding member 72 than the supporting point S3 of the swing member 76 or on the side closer to the proximal portion 72b of the first holding member 72 than the supporting point S3 of the swing member 76.

The protrusion 88 is provided on the side of the first and second holding surfaces 80 and 82 closer to the distal portion 72a of the first holding member 72 than the supporting point S3 of the swing member 76 when the limiting portion 78 is provided on the side closer to the distal portion 72a of the first holding member 72 than the supporting point S3 of the swing member 76. The protrusion 88 is provided on the side of the first and second holding surfaces 80 and 82 closer to the proximal portion 72b of the first holding member 72 than the supporting point S3 of the swing member 76 when the limiting portion 78 is provided on the side closer to the proximal portion 72b of the first holding member 72 than the supporting point S3 of the swing member 76.

In this embodiment, the cutter 54 and the cutter guide grooves 152 and 154 are not always necessary. The high-frequency electrodes 84 and 86 are not exclusively substantially U-shaped, and can be set to a suitable shape.

Next, the second embodiment is described with reference to FIG. 8A to FIG. 12B. This embodiment is a modification of the first embodiment. The same components as the components described in the first embodiment or the components having the same functions are provided with the same signs as much as possible, and detailed explanations thereof are omitted.

As in the first embodiment, the treatment instrument 12 of the curative treatment system 10 according to this embodiment includes the handle 22, the shaft 24 having the central axis C, and the treatment portion 26 (see FIG. 1). In this embodiment, the part between one end 22a of the handle 22 and the proximal end thereof where the cable 28 extends is a main body 22c (see FIG. 8A and FIG. 8B).

The other end 22b of the handle 22 is a grasp portion to be grasped by the surgeon. As shown in FIG. 8A and FIG. 8B, the handle 22 includes a treatment portion open-close knob (first operation body) 232 provided in parallel with the other end 22b.

The treatment portion open-close knob 232 has a pivot shaft 312, an operation portion 314, a protective portion 316, and a pair of claws 318. The treatment portion open-close knob 232 is rotatable by the pivot shaft 312 inside the handle 22. The operation portion 314 and the protective portion 316 protrude downward from the lower end of the main body 22c of the handle 22, and are disposed in front of the other end 22b of the handle 22. Thus, the operation portion 314 can be brought closer to (see FIG. 8B) or away from (see FIG. 8A) the other end 22b of the handle 22. The protective portion 316 is formed integrally with the operation portion 314 forward of the operation portion 314 (on the side close to the treatment portion 26). Thus, the protective portion 316 functions as an interlocking portion which moves together with the operation portion 314 as the first operation body. The pair of claws 318 are located at the upper end of the treatment portion open-close knob 232, substantially Y-shaped to support the proximal end of the internal cylinder 42, and engaged with a pair of pins 42a disposed at the proximal end of the internal cylinder 42 of the shaft 24 inside the main body 22c of the handle 22. The pair of pins 42a extend to the outer circumferential surface of the internal cylinder 42 in an outward direction to intersect at right angles with the central axis C. Thus, the pins 42a disposed at the proximal end of the internal cylinder 42 are supported movably relative to the claws 318 at the upper end of the treatment portion open-close knob 232. Thus, the internal cylinder 42 is movable relative to the handle 22 and the internal cylinder 42 by the operation of the operation portion 314 of the treatment portion open-close knob 232. That is, in this embodiment, the proximal end of the external cylinder 44 is fixed to the handle 22, in contrast to the structure according to the first embodiment.

The handle 22 further includes a cutter driving knob (second operation body) 234 provided forward of and beside the treatment portion open-close knob 232 to move the later-described cutter 54.

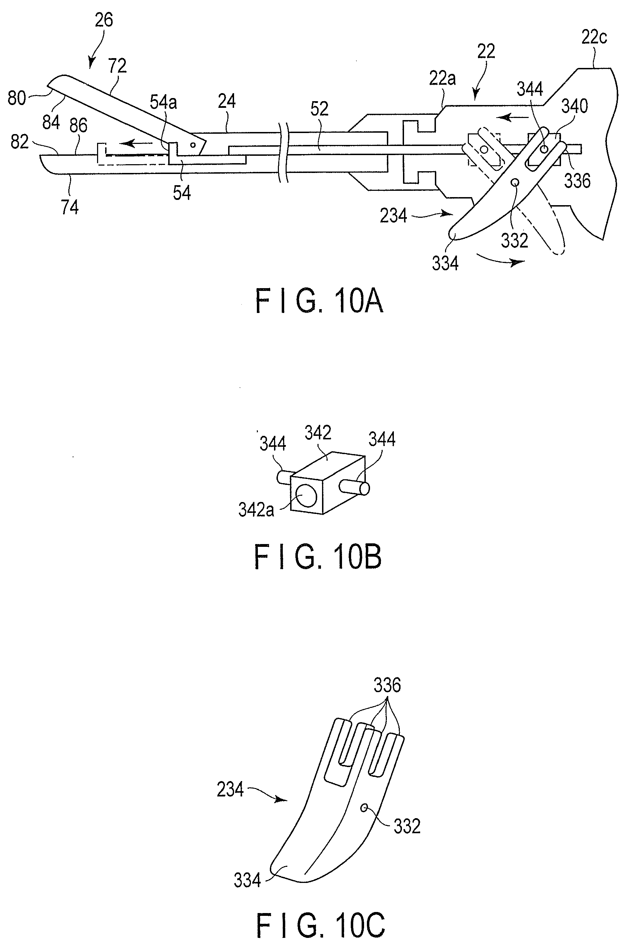

As shown in FIG. 10A and FIG. 10C, the cutter driving knob 234 has a pivot support portion 332, an operation portion 334, and a claw 336. The cutter driving knob 234 is rotatable by the pivot support portion 332 inside the handle 22. The operation portion 334 of the cutter driving knob 234 protrudes outward from the lower end of the main body 22c of the handle 22, and is disposed in front of the other end 22b of the handle 22. Thus, the operation portion 334 of the cutter driving knob 234 can be brought closer to or away from the other end 22b of the handle 22. The claw 336 is located at the upper end of the cutter driving knob 234, and engaged with a pin 344 provided in a coupling member 340 fixed to the proximal end of the drive rod 52 inside the main body 22c of the handle 22.

As shown in FIG. 10B, the coupling member 340 has a main body 342 having a through-hole 342a provided at the proximal end of the drive rod 52, and the pin 344 extending in an outward direction that intersects at right angles with the central axis C of the through-hole 342a. The coupling member 340 is fixed to the proximal end of the drive rod 52. As shown in FIG. 10A, the pin 344 extends in a direction that intersects at right angles with the plane of the cutter 54, and is engaged with the claw 336 located at the upper end of the cutter driving knob 234.

Thus, the operation portion 334 of the cutter driving knob 234 can be brought closer to or away from the other end 22b of the handle 22. The operation portion 334 of the cutter driving knob 234 is operated, so that the cutter 54 advances if the drive rod 52 is advanced via the coupling member 340, or the cutter 54 retreats if the drive rod 52 is retreated via the coupling member 340. At the same time, the cutter 54 moves along the later-described first and second cutter guide grooves (flow paths, fluid discharge grooves) 152 and 154. Particularly the distal end of the cutter 54 is located slightly closer to the proximal side than the distal ends of the cutter guide grooves 152 and 154 when the distal end of the cutter 54 has most advanced. The distal end of the cutter 54 is set to be located inside the distal end of the internal cylinder 42 or located at the positions of the proximal ends of the cutter guide grooves 152 and 154 without contacting the living tissue when the distal end of the cutter 54 has most retreated.

The protective portion 316 of the treatment portion open-close knob 232 is formed so that the fingers of the grasping hand may not contact the operation portion 334 of the cutter driving knob 234 while the operation portion 314 of the treatment portion open-close knob 232 is located away from the other end 22b of the handle 22. For example, as shown in FIG. 9A, a surface 318a which hides the operation portion 334 of the cutter driving knob 234 to prevent visual recognition thereof is formed in the front surface of the protective portion 316 of the treatment portion open-close knob 232. A modification of the protective portion 316 is shown in FIG. 9B. As shown in FIG. 9B, in the front surface of the protective portion 316 of the treatment portion open-close knob 232, a pair of ends 318b are formed to permit the visual recognition of the operation portion 334 of the cutter driving knob 234 but to prevent the fingers from contacting the operation portion 334 of the cutter driving knob 234. The space between the pair of ends 318b is formed to be smaller than the thickness of the finger.

As shown in FIG. 8A and FIG. 8B, a push-out surface 316a is formed in the protective portion 316 of the treatment portion open-close knob 232. The push-out surface 316a pushes the operation portion 334 of the cutter driving knob 234 away from the other end 22b of the handle 22 if the operation portion 314 of the treatment portion open-close knob 232 is brought away from the other end 22b of the handle 22 when the operation portion 334 of the later-described cutter driving knob 234 is located close to the other end 22b of the handle 22.

As shown in FIG. 10A, the treatment portion 26 according to this embodiment has the first and second holding members 72 and 74, the first and second holding surfaces 80 and 82, the first and second energy applying portions (high-frequency electrodes) 84 and 86, and the protrusion 88. In this embodiment, the swing member 76 described in the first embodiment is not used, and the first holding member 72 has the first holding surface 80, and the first energy applying portion 84 is provided in the first holding surface 80.

The first holding surface 80 which cooperates with the second holding surface 82 to grasp the living tissue is provided on the side of the first holding member 72 closer to the second holding member 74.

The thin plate-shaped first high-frequency electrode (first energy applying portion) 84 which applies high-frequency energy to the living tissue grasped between the first and second holding surfaces 80 and 82 to generate heat in the living tissue is fixed to the first holding surface 80. The surface of the first high-frequency electrode 84 is substantially U-shaped, and cooperates with the first holding surface 80 to form the first cutter guide groove (treatment assist tool guide groove) 152 to guide the cutter 54. That is, the first holding surface 80 and the first high-frequency electrode 84 have the linear first cutter guide groove (treatment assist tool guide groove) 152 at a position along the central axis C.

The structure of the first holding member 72 to open and close relative to the second holding member 74 is the same as the structure described in the first embodiment (see FIG. 2A and FIG. 2B) and is therefore not described. The structure in which the second holding surface 82, the second high-frequency electrode (second energy applying portion) 86, and the second cutter guide groove 154 are provided in the second holding member 74 is the same as the structure described in the first embodiment and is therefore not described.

Therefore, the treatment portion 26 according to this embodiment has the structure described below.

The first and second holding members 72 and 74 of the treatment portion 26 are openable and closable relative to each other. The cutter (treatment assist tool) 54 is movable between the position located between the first and second holding members 72 and 74 and the position to escape from the position located between the first and second holding members 72 and 74. The first operation body (treatment portion open-close knob) 232 can operate the opening and closing of the first and second holding members 72 and 74. The second operation body (cutter driving knob) 234 can operate the movement of the cutter 54. The protective portion (interlocking portion) 316 moves together with the first operation body 232 to limit the operation of the second operation body 234 when the first operation body 232 opens the first and second holding members 72 and 74, and to permit the operation of the second operation body 234 when the first operation body 232 closes the first and second holding members 72 and 74.

The second operation body 234 is located closer to the first and second holding members 72 and 74 than the first operation body 232.

The interlocking portion 316 has a protective portion which covers at least part of the second operation body 234 when the first operation body 232 opens the first and second holding members 72 and 74 relative to each other and which releases the second operation body 234 when the first operation body 232 has closed the first and second holding members 72 and 74.

Next, the function of the curative treatment system 10 according to this embodiment is described. The mechanism which opens and closes the first and second holding members 72 and 74 is the same as the mechanism described in the first embodiment, thus the mechanism which opens and closes the first and second holding members 72 and 74 is described with reference to FIG. 2A and FIG. 2B.

As shown in FIG. 8B, from the condition in which the operation portion 314 of the treatment portion open-close knob 232 is brought closer to the handle 22 and the internal cylinder 42 is retreated relative to the external cylinder 44 as shown in FIG. 2B, the internal cylinder 42 is advanced relative to the external cylinder 44 as shown in FIG. 2A. Thus, the first holding member 72 is closed relative to the second holding member 74. While the treatment portion 26 is closed, the treatment portion 26 is inserted into, for example, a body cavity. At the same time, the protective portion 316 of the treatment portion open-close knob 232 separates from the lower end of the main body 22c of the handle 22, and exposes the cutter driving knob 234. Thus, the cutter driving knob 234 is operable, but the cutter driving knob 234 is not operated.

The operation portion 314 of the treatment portion open-close knob 232 is brought away from the handle 22 as shown in FIG. 8A while the closed first and second holding members 72 and 74 face the living tissue to be treated. The internal cylinder 42 is then retreated relative to the external cylinder 44 as shown in FIG. 2B from the condition in which the internal cylinder 42 has advanced relative to the external cylinder 44 as shown in FIG. 2A. Thus, the first holding member 72 is opened relative to the second holding member 74.

At the same time, as shown in FIG. 8A, FIG. 9A, and FIG. 9B, the protective portion 316 of the treatment portion open-close knob 232 moves closer to the lower end of the main body 22c of the handle 22, and covers the cutter driving knob 234. That is, the front side of the operation portion 334 of the cutter driving knob 234 is protected by the protective portion 316 of the treatment portion open-close knob 232. Thus, the operation of the cutter driving knob 234 is prevented, and the movement of the cutter 54 from the proximal ends of the cutter guide grooves 152 and 154 to the distal ends is prevented.

The operation portion 334 of the cutter driving knob 234 is pushed away from the other end 22b of the handle 22 by the push-out surface 316a of the protective portion 316 of the treatment portion open-close knob 232 in response to the opening of the first holding member 72 relative to the second holding member 74 when the edge 54a of the cutter 54 is located closer to the distal side than the proximal ends of the cutter guide grooves 152 and 154. Thus, in response to the opening of the first holding member 72 relative to the second holding member 74, the edge 54a of the cutter 54 can be moved towards the proximal ends of the cutter guide grooves 152 and 154.

There is a slight space between the lower end of the main body 22c of the handle 22 and the protective portion 316 of the treatment portion open-close knob 232, and the fingers of the hand grasping the handle 22 do not reach the operation portion 334 of the cutter driving knob 234.

In this condition, the living tissue to be treated is disposed between the first and second holding surfaces 80 and 82, that is, between the first and second high-frequency electrodes 84 and 86. In this condition, the treatment portion open-close knob 232 located before the handle 22 is moved forward, and the first holding member 72 is closed relative to the second holding member 74. That is, the living tissue to be treated is grasped between the first and second high-frequency electrodes 84 and 86. At the same time, the protective portion 316 can operate the cutter driving knob 234.

The operation portion 334 of the cutter driving knob 234 is not yet operated toward the other end 22b of the handle 22, and the pedal 16a of the foot switch 16 is depressed by the foot to apply high-frequency energy to the living tissue between the first and second high-frequency electrodes 84 and 86 from the energy source 14 to generate heat in the living tissue and then coagulate the living tissue. At the same time, the living tissue is continuously sealed substantially in a U-shape.

After the foot is taken off pedal 16a of the foot switch 16, the operation portion 334 of the cutter driving knob 234 is operated toward the other end 22b of the handle 22, and the treated living tissue is cut with the cutter 54.

The operation portion 314 of the treatment portion open-close knob 232 is moved away from the other end 22b of the handle 22 when the treated living tissue is released from the space between the first and second high-frequency electrodes 84 and 86. At the same time, the operation portion 334 of the cutter driving knob 234 is pushed forward (to the side closer to the treatment portion 26) by the push-out surface 316a of the protective portion 316. Therefore, in response to the release of the treated living tissue from the space between the first and second high-frequency electrodes 84 and 86, the edge 54a of the cutter 54 retreats to the inside of the distal end of the internal cylinder 42 where the edge 54a does not contact the living tissue, or to the position of the proximal ends of the cutter guide grooves 152 and 154.

As described above, the following can be said according to this embodiment.

In the treatment instrument 12 according to this embodiment, the cutter driving knob 234 can be protected by the protective portion 316 of the treatment portion open-close knob 232 when the first and second holding members 72 and 74 are opened. Thus, the advance of the cutter 54 can be prevented while the first and second holding members 72 and 74 are open. On the other hand, when the first and second holding members 72 and 74 are closed, the protective portion 316 of the treatment portion open-close knob 232 evacuates to the position where the cutter driving knob 234 is exposed (see FIG. 8B) from the position where the cutter driving knob 234 is protected (see FIG. 8A), so that the operation portion 334 of the cutter driving knob 234 can be operated.

Therefore, it is possible to prevent the cutter 54 from being disposed between the first and second holding surfaces 80 and 82, that is, between the first and second high-frequency electrodes 84 and 86 when the first and second holding members 72 and 74 are open. Therefore, in the treatment instrument 12 according to this embodiment, erroneous operations by the user can be prevented regarding the operation of the cutter 54, and the operability of the treatment instrument 12 can be improved.

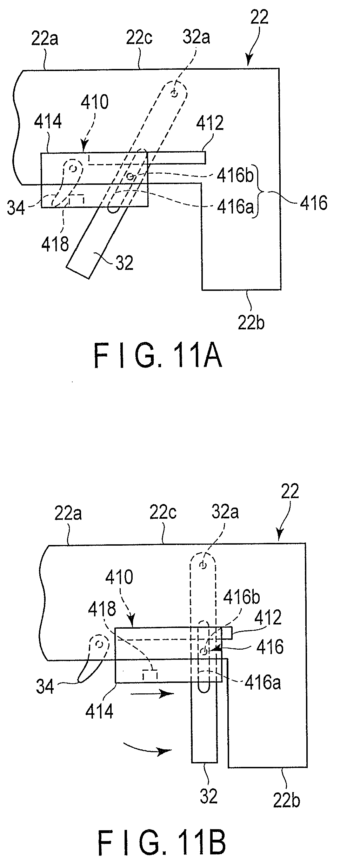

Next, a first modification of the second embodiment is described with reference to FIG. 11A and FIG. 11B.

As shown in FIG. 11A and FIG. 11B, the treatment portion open-close knob according to this embodiment is formed in a manner similar to the treatment portion open-close knob 32 shown in FIG. 1 according to the first embodiment. The treatment portion open-close knob 32 is rotatably supported by a pivot shaft 32a inside the main body 22c of the handle 22. A slide mechanism 410 to protect the cutter driving knob 34 is connected to the treatment portion open-close knob 32.

The slide mechanism 410 has a rail 412 provided at the lower end of the main body 22c of the handle 22, a slider (link portion) 414 movable along the rail 412, and a coupling portion 416 which couples the slider 414 to the treatment portion open-close knob 32. The cross section of the slider 414 is substantially U-shaped.

A space is formed between the lower end of the main body 22c of the handle 22 and the lower end of the slider 414 of the slide mechanism 410, and the cutter driving knob 34 can be housed in this space. The lower end of the treatment portion open-close knob 32 protrudes downward relative to the slider 414 of the slide mechanism 410, so that the treatment portion open-close knob 32 is operable. The slider 414 has a push surface 418 therein which pushes the cutter driving knob 34 and retreats the edge 54a of the cutter 54 from the first and second cutter guide grooves 152 and 154 when the treatment portion open-close knob 32 is brought away from the other end 22b of the handle 22.

The coupling portion 416 has a long hole 416a formed along the longitudinal direction of the treatment portion open-close knob 32, and an action pin 416b provided in the slider 414.

The function of the treatment instrument 12 according to this modification is described.

The treatment portion open-close knob 32 is brought away from the other end 22b of the handle 22 as shown in FIG. 11A from the condition in which the treatment portion open-close knob 32 is located close to the other end 22b of the handle 22 as shown in FIG. 11B. That is, the first holding member 72 is opened relative to the second holding member 74. At the same time, the action pin 416b of the slider 414 is pushed forward by the edge of the long hole 416a of the treatment portion open-close knob 32 in accordance with the operation of the treatment portion open-close knob 32. Thus, the slider 414 moves forward along the rail 412. The push surface 418 advances in response to the advance of the slider 414, so that the edge 54a of the cutter 54 is retreated by the position of the cutter driving knob 34. Thus, the cutter driving knob 34 is housed in the slider 414 on its rear side rather than its distal side. Therefore, the slider 414 functions as a protective portion which protects the cutter driving knob 34 from operation at the position where the treatment portion open-close knob 32 is brought away. Thus, the slider 414 functions as an interlocking portion which moves together with the treatment portion open-close knob 32 as the first operation body.

On the other hand, the treatment portion open-close knob 32 is brought closer to the other end 22b of the handle 22 as shown in FIG. 11B from the condition in which the treatment portion open-close knob 32 is located away from the other end 22b of the handle 22 as shown in FIG. 11A. That is, the first holding member 72 is closed relative to the second holding member 74. At the same time, the action pin 416b of the slider 414 is pushed backward by the edge of the long hole 416a of the treatment portion open-close knob 32 in accordance with the operation of the treatment portion open-close knob 32. Thus, the slider 414 retreats along the rail 412. Thus, the cutter driving knob 34 is disposed closer to the distal side than the distal end of the slider 414. At the same time, the cutter driving knob 34 is freely operable.

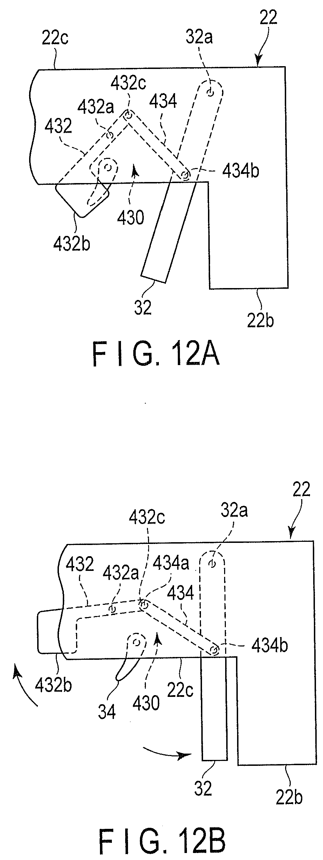

Next, a second modification of the second embodiment is described with reference to FIG. 12A and FIG. 12B.

As shown in FIG. 12A and FIG. 12B, a link mechanism 430 to protect the cutter driving knob 34 is connected to the treatment portion open-close knob 32.

The link mechanism 430 has a first link (link portion) 432 pivotally supported by the main body 22c of the handle 22, and a second link (link portion) 434 having one end supported by the treatment portion open-close knob 32 and the other end supported by the other end of the first link 432.

The first link 432 has a central supporting point 432a supported by the main body 22c of the handle 22, a protective portion 432b, and a first connection end 432c for the second link 434. The second link 434 has a second connection end 434a for the first connection end 432c of the first link 432, and a third connection end 434b for the treatment portion open-close knob 32. The protective portion 432b at one end of the first link 432 is disposed to be able to protect the cutter driving knob 34, that is, limit the operation of the cutter driving knob 34.

The function of the treatment instrument 12 according to this modification is described.

The treatment portion open-close knob 32 is brought away from the other end 22b of the handle 22 as shown in FIG. 12A from the condition in which the treatment portion open-close knob 32 is located close to the other end 22b of the handle 22 as shown in FIG. 12B. That is, the first holding member 72 is opened relative to the second holding member 74. At the same time, the second connection end 434a in the second link 434 to the first link 432 moves upward in accordance with the operation of the treatment portion open-close knob 32. Thus, the first connection end 432c in the first link 432 to the second link 434 moves forward. Since the first link 432 rotates around the axis of the central supporting point 432a, the protective portion 432b which has evacuated to the front side and upper side of the cutter driving knob 34 moves downward, and then holds the cutter driving knob 34. Therefore, the protective portion 432b protects the cutter driving knob 34 from operation. Thus, the first and second links 432 and 434 function as interlocking portions which move together with the operation of the treatment portion open-close knob 32 as the first operation body.

On the other hand, the treatment portion open-close knob 32 is brought closer to the other end 22b of the handle 22 as shown in FIG. 12B from the condition in which the treatment portion open-close knob 32 is located away from the other end 22b of the handle 22 as shown in FIG. 12A. That is, the first holding member 72 is closed relative to the second holding member 74. At the same time, the protective portion 432b performs an operation reverse to the above-described operation, and thus evacuates to the front and upper sides of the cutter driving knob 34 in accordance with the operation of the treatment portion open-close knob 32. At the same time, the cutter driving knob 34 is freely operable.

Therefore, according to the first and second modifications of the second embodiment, the interlocking portions 414, 432, and 434 have link portions which move relative to the handle main body 22c when moving together with the first operation body 32.

Although the first holding surface 80 and the first high-frequency electrode 84 are disposed in the first holding member 72 in the treatment portion 26 according to the second embodiment including the first and second modifications in the described examples, it should be understood that the swing member 76 may be pivotally supported on the first holding member 72 of the treatment portion 26 as has been described in the first embodiment. That is, when the treatment portion open-close knob 232, 32 according to this embodiment is disposed in the handle 22 of the treatment instrument 12 and the cutter driving knob 234, 34 is disposed, it is also preferable that what is known as a seesaw jaw is used for the treatment portion 26.

Although the first and second high-frequency electrodes 84 and 86 in the treatment instrument 12 described according to the first and second embodiments including the modifications are bipolar types, the first and second high-frequency electrodes 84 and 86 may be monopolar types which are used so that an unshown return electrode is attached to a patient.

Although the treatment instrument 12 in which the thin plate-shaped high-frequency electrodes 84 and 86 are respectively disposed in the holding surfaces 80 and 82 is described in the examples according to the first and second embodiments including the modifications, thin plate-shaped heaters (energy applying portions) may be used instead of the high-frequency electrodes 84 and 86. Alternatively, thin plate-shaped heaters disposed on the rear surfaces of the thin plate-shaped high-frequency electrodes 84 and 86 may be used.

Although the cutter driving knob 34 is operated to advance or retreat the cutter 54 in the examples described according to the first and second embodiments including the modifications, the treatment assist tool is not limited to the cutter as long as the treatment assist tool is used to assist in a treatment. For example, an ultrasonically vibrating probe may be used instead of the cutter 54.

While some embodiments have been described in detail with reference to the drawings so far, this invention is not limited to the embodiments described above, and covers all embodiments carried out without departing from the spirit thereof.

## Claims

1. A treatment instrument configured to treat a living tissue by applying energy thereto, the treatment instrument comprising:
first and second holding members openable and closable relative to each other, each of first and second holding members including a distal portion, a proximal portion, and a longitudinal axis defined by the distal portion and the proximal portion;
a swing member supported between the distal portion and the proximal portion of the first holding member, the swing member being rotatable in a first direction and in a second direction opposite to the first direction on a rotation axis as a supporting point which extends in a direction perpendicular to the longitudinal axis and in a direction that intersects at right angles with an open-close direction of the first and second holding members;
a limiting portion provided in at least one of the first holding member and the swing member and which limits the rotation of the swing member in one of the first direction and the second direction;
a first holding surface which is provided on the side of the swing member close to the second holding member and which is configured to hold the living tissue;
a second holding surface which is provided on the side of the second holding member close to the swing member and which faces the first holding surface and which cooperates with the first holding surface of the swing member to hold the living tissue;
an energy applying portion which is provided in at least one of the first and second holding surfaces and which applies energy to the living tissue held between the first and second holding surfaces; and
a protrusion having heat resisting properties and electric insulating properties which is provided in at least one of the first and second holding surfaces and which forms a clearance between the first and second holding surfaces when the first and second holding surfaces are closed relative to each other.

2. The treatment instrument according to claim 1,
wherein the protrusion faces the limiting portion across the swing member when the first and second holding surfaces are closed relative to each other.

3. The treatment instrument according to claim 1,
wherein the limiting portion is provided on the side closer to the distal portion of the first holding member than the supporting point of the swing member or on the side closer to the proximal portion of the first holding member than the supporting point of the swing member.

4. The treatment instrument according to claim 3,
wherein
the protrusion is provided on the side of the first and second holding surfaces closer to the distal portion of the first holding member than the supporting point of the swing member when the limiting portion is provided on the side closer to the distal portion of the first holding member than the supporting point of the swing member, and
the protrusion is provided on the side of the first and second holding surfaces closer to the proximal portion of the first holding member than the supporting point of the swing member when the limiting portion is provided on the side closer to the proximal portion of the first holding member than the supporting point of the swing member.

5. The treatment instrument according to claim 1,
wherein
the first holding surface has a first guide groove to guide a cutter configured to cut the living tissue held between the first and second holding surfaces,
the second holding surface has a second guide groove which cooperates with the first guide groove to guide the cutter, and
the protrusion is located adjacent to at least one of the first and second guide grooves.

6. The treatment instrument according to claim 1,
wherein
the first holding surface has a first guide groove to guide a cutter configured to cut the living tissue held between the first and second holding surfaces,
the second holding surface has a second guide groove which cooperates with the first guide groove to guide the cutter,
the energy applying portion has its surface substantially U-shaped to have a guide groove which cooperates with at least one of the first and second holding surfaces to guide the cutter, and
the protrusion is located at a position in the substantially U-shaped energy applying portion that does not prevent the seamless and continuous sealing of the living tissue.

7. The treatment instrument according to claim 6,
wherein
the protrusion is provided on the side of the first and second holding surfaces closer to the distal portion of the first holding member than the supporting point of the swing member when the limiting portion is provided on the side closer to the distal portion of the first holding member than the supporting point of the swing member, and
the protrusion is provided on the side of the first and second holding surfaces closer to the proximal portion of the first holding member than the supporting point of the swing member when the limiting portion is provided on the side closer to the proximal portion of the first holding member than the supporting point of the swing member.

8. A treatment instrument configured to treat a living tissue by applying energy thereto, the treatment instrument comprising:
first and second holding members openable and closable relative to each other;
a treatment assist tool movable between the position located between the first and second holding members and the position to escape from the position located between the first and second holding members;
a first operation body configured to operate the opening and closing of the first and second holding members;
a second operation body configured to operate the movement of the treatment assist tool; and
an interlocking portion which moves together with the first operation body to limit the operation of the second operation body when the first and second holding members are opened relative to each other by the operation of the first operation body.

9. The treatment instrument according to claim 8,
wherein the second operation body is located closer to the first and second holding members than the first operation body.

10. The treatment instrument according to claim 8,
wherein the interlocking portion has a protective portion which covers at least part of the second operation body when the first and second holding members are opened relative to each other by the operation of the first operation body and which releases the second operation body when the first and second holding members are closed by the operation of the first operation body.

11. The treatment instrument according to claim 8, further comprising a handle main body in which the first and second operation bodies are provided,
wherein the interlocking portion has a link portion which moves relative to the handle main body when moving together with the first operation body.

12. The treatment instrument according to claim 11, wherein the link portion includes:
a rail provided in the handle main body, and
a slider which moves along the rail together with the operation of the first operation body and which limits the operation of the second operation body when the first and second holding members are opened by the operation of the first operation body.

13. The treatment instrument according to claim 12,
wherein the link portion further includes a coupling portion which couples the slider to the first operation body.

14. The treatment instrument according to claim 11, wherein the link portion includes:
a first link pivotally supported by the handle main body, and
a second link having one end supported by the first operation body and the other end supported by the first link.

15. The treatment instrument according to claim 14, wherein the first link includes:
a central supporting point supported by the handle main body,
a first connection end with respect to the second link, and
a protective portion which moves together with the operation of the first operation body and which limits the operation of the second operation body when the first and second holding members are opened by the operation of the first operation body.

16. The treatment instrument according to claim 15, wherein the second link includes:
a second connection end with respect to the first connection end of the first link, and
a third connection end with respect to the first operation body.
